# EUROPEAN PATENT SPECIFICATION

(11) **EP 0 705 318 B1**
(45) Date of publication and mention of the grant of the patent: **16.04.1997**
(21) Application number: 93913240.3
(22) Date of filing: 28.05.1993
(51) Int. Cl.: C09K 19/20, C09K 19/46, C07C 69/94

(54) **LIQUID CRYSTAL COMPOUNDS, MIXTURES AND DEVICES**
FLÜSSIGKRISTALLVERBINDUNGEN, -MISCHUNGEN UND -VORRICHTUNGEN
COMPOSES, MELANGES ET DISPOSITIFS A CRISTAUX LIQUIDES

(30) Priority: 10.06.1992 GB 9212334
(43) Date of publication of application: 10.04.1996
(73) Proprietor: SECRETARY OF STATE FOR DEFENCE IN HER BRITANNIC MAJESTY'S GOV. OF THE UNITED KINGDOM OF GREAT BRITAIN AND NORTHERN IRELAND, Farnborough, Hants. GU14 6TD (GB)
(72) Inventor: GOODBY, John William, Hull HU6 7RX (GB); TOYNE, Kenneth Johnson, Hull HU6 7RX (GB); BOOTH, Christopher James, Hull HU6 7RX (GB)
(74) Representative: Beckham, Robert William
(86) International application number: PCT/GB93/01133
(87) International publication number: WO 93/25631

(56) References cited:
- EP-A- 0 197 677
- FR-A- 2 612 182
- LIQUID CRYSTALS vol. 11, no. 1, January 1992, LONDON GB pages 135 - 143 J .W. GOODBY ET AL. 'the effect of structural changes in the molecular core and periphery on the liquid-crystalline properties of some chiral 4-n- alkoxyphenylpropiolates' s

## Description

This invention relates to liquid crystal compounds, liquid crystal materials containing them and their inclusion in liquid crystal devices.

Liquid crystals can exist in various phases. In essence there are three different classes of liquid crystalline material, each possessing a characteristic molecular arrangement. These classes are nematic, cholesteric and smectic. A wide range of smectic phases exists, for example smectic A and smectic C. Some liquid crystal materials possess a number of liquid crystal phases on varying the temperature, others have just one phase. For example, a liquid crystal material may show the following phases on being cooled from the isotropic phase:- isotropic - nematic - smectic A - smectic C - solid. If a material is described as being smectic A then it means that the material possesses a smectic A phase over a useful working temperature range.

Compounds such as: 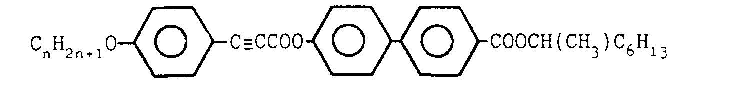 are described in J. Am. Chem. Soc., 1989, 111, 8119-8125 by J.W. Goodby *et. al*., where n ranges from 8 to 16. At long *n*-alkoxy chain lengths the series exhibits a novel variation of mesophase behaviour to which a smectic A* phase was assigned. Some of the materials also appeared to exhibit two further mesophases.

According to this invention there are provided compounds having a general formula I 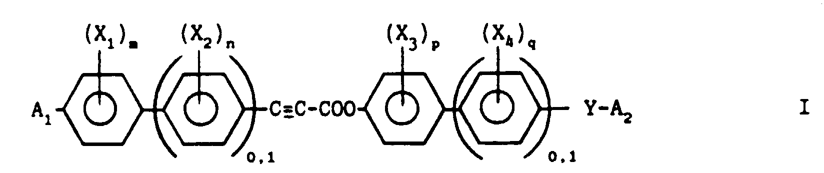 in which A₁ is selected from alkyl, alkoxy or alkenyl; X₁, X₂, X₃ and X₄ are independently selected from the halogen group; m, n, p and q are independently 0, 1, 2, 3 or 4 such that m+n+p+q ≠ 0; Y is selected from 0 and COO; A₂ is an end group of formula II 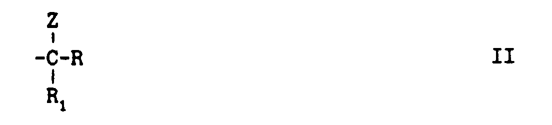 where Z is selected from halogen, CH₃, CN, CF₃, CHF₂; R is a linear or branched alkyl group containing 2-15 carbon atoms or H; R₁ is a linear or branched alkyl group containing 1-5 carbon atoms or H.

A₁ is C₃₋₂₀, preferably C₆₋₁₆, more preferably C₉₋₁₅.

Preferably A₂ is a chiral end group.

Preferably R is C₂₋₁₅, more preferably C₃₋₁₀, even more preferably C₅₋₇.

Preferably R₁ is H.

Preferably Z is CH₃ or CN.

Preferably X is F.

Preferably each of m, n, p and/or q is independently 0, 1 or 2 such that m+n+p+q = 1 or 2.

Compounds of Formula I can be included in a material, the material being a mixture of compounds.

The materials of this aspect of the invention may be used in many of the known forms of liquid crystal display devices, for example chiral smectic electrooptic devices. Such a device may comprise a layer of liquid crystal material contained between two spaced cell walls bearing electrode structures and surface treated to align liquid crystal material molecules. The liquid crystal mixtures may have applications in ferroelectric, ferrielectric (M. Johno *et. al.,* Japan Display, 1989, 22), antiferroelectric, thermochromic and electroclinic devices; they may also lead to the formation of frustrated liquid crystal phases. Frustrated phases arise from mesophases possessing double twist structures which means that space cannot be filled uniformly and the phase is stabilised via a lattice of defects. Frustrated phases can also arise from the competition between helical and layer structures, an example of which is the smectic A* analogue of the Abrikosov flux phase as described by J. W. Goodby, M. A. Waugh, S. M. Stein, E. Chin, R. Pindak, Nature, 1989, 337, 449; A. J. Slaney and J. W. Goodby, J. Mater. Chem, 1991, 1, 5. The competition between helical and layer structures leads to defects being formed which again stabilise the phase.

Ferroelectric smectic liquid crystal materials, which can be produced by mixing an achiral host and a chiral dopant, use the ferroelectric properties of the tilted chiral smectic C, F, G, H, I, J and K phases. The chiral smectic C phase is denoted S_{c}* with the asterisk denoting chirality. The S_{c} phase is generally considered to be the most useful as it is the least viscous. Ferroelectric smectic liquid crystal materials should ideally posses the following characteristics: low viscosity, controllable spontaneous polarisation (Ps) and an S_{c} phase that persists over a broad temperature range, which should include ambient temperature and exhibits chemical and photochemical stability. Materials which possess these characteristics offer the prospect of very fast switching liquid crystal containing devices. Some applications of ferroelectric liquid crystals are described by J.S. Patel and J.W. Goodby in Opt. Eng., 1987, 26, 273.

The electroclinic effect, first described by S. Garoff and R. Meyer, Phys. Rev. Lett., 38, 848, 1977, usually occurs in the smectic A phase. Unlike ferroelectric devices, the liquid crystal material in electroclinic devices is not bistable. The liquid crystal director within an EC device responds almost linearly to an applied electric field. Electroclinic devices are suitable for various applications including spatial light modulators.

Chandani *et al*., Jpn. J. Appl. Phys., 27, L 729, 1988; Jpn. J. Appl. Phys., 28, L 1261, 1989; Jpn. J. Appl. Phys., 28, L 1265, 1989, first described the antiferroelectric effect which is a tri-stable switching state occurring in a liquid crystal phase designated as SmC_{A}*. For example, when ferroelectric layers are stacked so that the polarisation vectors in sequential layers oppose one another then an antiferroelectric phase is obtained.

For a review of thermochromism in liquid crystals see J.G. Grabmaier in Applications of Liquid Crystals', G. Meier, E. Sackmann and J.G. Grabmaier, Springer-Verlag, Berlin and New York, 1975, pp 83-158.

For all the above applications it is not usual for a single compound to exhibit all of the properties highlighted, for example ferroelectric smectic liquid crystal materials generally consist of a mixture of compounds which when mixed together induce a chiral tilted smectic phase. Chiral dopants are added to a liquid crystalline mixture in order to induce the smectic mixture to become chiral smectic and to induce a Ps in the material, or if the material already possesses a Ps then the introduction of a chiral dopant should result in a change of value for Ps.

The invention will now be described by way of example only with reference to the accompanying drawings of which:-

**Figure 1** describes a synthetic route for the preparation of example compounds **46** to **65,** *i.e.* compounds of general formula VII where X and Y are described in Tables 5 and 6.

**Figure 2** illustrates a liquid crystal device.

**Figures 3-12** are phase diagrams for compounds **46-65** (*R*) and (*S*) mixtures.

**Figures 13 and 14** are graphs of optical tilt angle/° versus reduced temperature/°C for compounds **52** and **56** respectively.

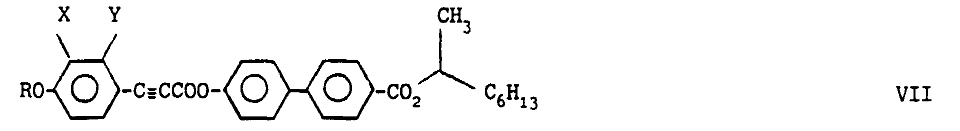

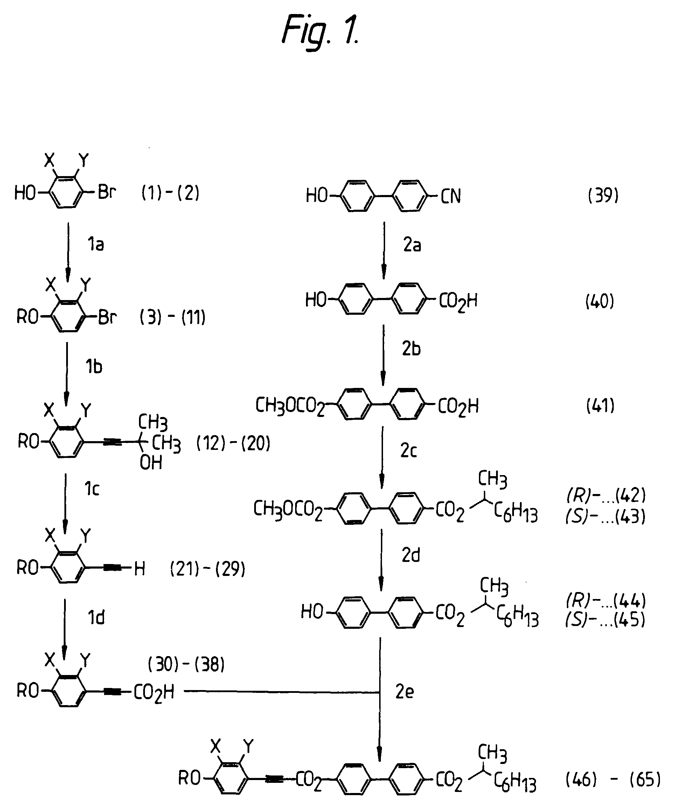

Reagents used in the synthetic route of Figure 1 are shown in Route 1.

Compound **1** is synthesised from 3-fluorophenol (Fluorochem Ltd) by a fast bromination at -5°C (Br₂, acetic acid). Compound **2** is commercially available from Fluorochem Ltd. Compound **39** is a commercially available liquid crystal precursor from Merck.

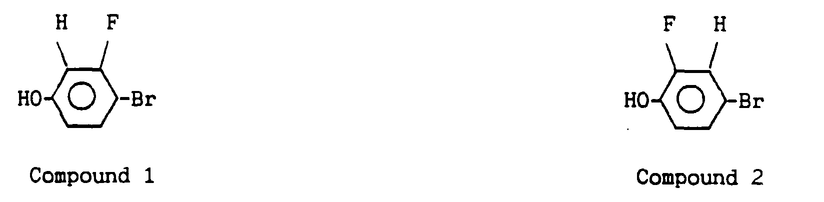

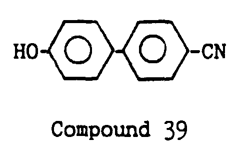

### Route 1

The steps are identified below:
- 1a: RBr, K₂CO₃, butanone, reflux.
- 1b: 3-methylbut-1-yn-3-ol, Pd(PPh₃)₄, CuI, (ⁱPr)₂NH, N₂, reflux.
- 1c: KOH, toluene, N₂, reflux.
- 1d: (i) BuLi, THF, N₂, -10°C; (ii) CO₂(s), THF, -10°C to RT; (iii) HCl (conc).
- 2a: H₂SO₄, AcOH, H₂O, reflux.
- 2b: (i) NaOH, CH₃OCOCl, H₂O, 0°C; (ii) 1:1 HCl:H₂O, pH=5.
- 2c: (R)- or (S)-2-octanol, diethyl azodicarboxylate, PPh₃, THF, N₂, RT.
- 2d: EtOH, NH₃ (aq), RT.
- 2e: Dicyclohexylcarbodiimide, 4-N,N-dimethylaminopyridine, CH₂Cl₂, RT.

This route allows the configuration about the chiral carbon to be easily controlled.

### Compound 3: Preparation of 1-Bromo-4-dodecyloxy-2-fluorobenzene

1-Bromodecane (12.01g, 48.2mmol) in butanone (20ml) was added dropwise to a stirred, refluxing mixture of 4-bromo-3-fluorophenol (**1**) (9.04g, 47.4mmol), potassium carbonate (10.12g, 73.2mmol) and butanone (100ml). The resulting reaction mixture was refluxed for a further 20 hours. The cooled reaction mixture was then filtered to remove the excess potassium carbonate and precipitated potassium bromide. The filtrate was washed with 5% (v/v) sodium hydroxide (2 x 50ml) then water (50 ml) and the organic layer then dried (MgSO₄), filtered and evaporated under reduced pressure to give a colourless oil which was purified by column chromatography [silica gel; 1:1 dichloromethane-petrol (bp 40-60°C)] to give a colourless liquid. This was then dried *in vacuo* (P₂O₅, 0.20mm Hg, RT, 5h).
Yield = 16.73g, (98%)

Compounds **3-11** have general formula III, see Table 1.

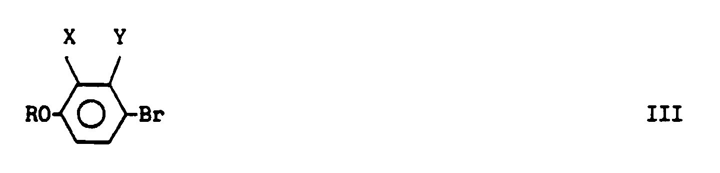

### Compound 4: Preparation of 1-Bromo-2-fluoro-4-tridecyloxybenzene

This was prepared using a similar method to that described for compound **3**. Quantities: compound **1** (4.02g, 21.1mmol), 1-bromotridecane (6.11g, 23.2mmol), potassium carbonate (5.22g, 37.8mmol) and butanone (115ml).
Yield = 6.62g, (84%)
mp = 26-27°C

### Compound 5: Preparation of 1-Bromo-2-fluoro-4-tetradecyloxybenzene

This was prepared using a similar method to that described for compound **3**. Quantities: compound **1** (4.03g, 21.1mmol), 1-bromotetradecane (6.41g, 23.1mmol), potassium carbonate (5.59g, 40.5mmol) and butanone (115ml).
Yield = 7.48g, (92%)
mp = 30-31°C

### Compound 6: Preparation of 1-Bromo-2-fluoro-4-pentadecyloxybenzene

This was prepared using a similar method to that described for compound **3**. Quantities: compound **1** (2.31g, 12.1mmol), 1-bromopentadecane (3.97g, 13.6mmol), potassium carbonate (2.97g, 21.5mmol) and butanone (80ml).
Yield = 4.35g, (90%)
mp = 32-36°C

### Compound 7: Preparation of 1-Bromo-4-dodecyloxy-3-fluorobenzene

This was prepared using a similar method to that described for compound **3**. Quantities: compound **2** (6.00g, 31.4mmol), 1-bromododecane (8.23g, 33.1mmol), potassium carbonate (5.67g, 40.9mmol) and butanone (100ml).
Yield = 11.11g, (99%)

### Compound 8: Preparation of 1-Bromo-3-fluoro-4-tridecyloxybenzene

This was prepared using a similar method to that described for compound **3**. Quantities: compound **2** (6.01g, 31.5mmol), 1-bromotridecane (8.75g, 33.2mmol), potassium carbonate (5.67g, 40.9mmol) and butanone (100ml).
Yield = 11.69g, (99%)
mp = 37-39°C

### Compound 9: Preparation of 1-Bromo-3-fluoro-4-tetradecyloxybenzene

This was prepared using a similar method to that described for compound **3**. Quantities: compound **2** (6.02g, 31.5mmol), 1-bromotetradecane (9.15g, 33.0mmol), potassium carbonate (5.67g, 40.9mmol) and butanone (100ml).
Yield = 11.82g, (97%)
mp = 28-29°C

### Compound 10: Preparation of 1-Bromo-3-fluoro-4-pentadecyloxybenzene

This was prepared using a similar method to that described for compound **3**. Quantities: compound **2** (5.04g, 26.4mmol), 1-bromotetradecane (8.15g, 27.9mmol), potassium carbonate (5.65g, 40.9mmol) and butanone (120ml).
Yield = 9.54g, (90%)
mp = 40-43°C

### Compound 11: Preparation of 1-Bromo-3-fluoro-4-hexadecyloxybenzene

This was prepared using a similar method to that described for compound **3**. Quantities: compound **2** (5.02g, 26.3mmol), 1-bromohexadecane (8.56g, 28.0mmol), potassium carbonate (5.19g, 37.6mmol) and butanone (100ml).
Yield = 10.09g, (92%)
mp = 41-43°C

### Compound 12: Preparation of 4-Dodecyloxy-2-fluoro-1-(3-hydroxy-3-methylbut-1-ynyl)benzene

A stream of dry nitrogen was bubbled through a stirred, dark green mixture of compound **3** (5.00g, 13.9mmol), tetrakis(triphenylphosphine) palladium (O) (1.02g, 0.89mmol), copper (I) iodide (0.11, 0.58mmol) and dry di-isopropylamine (30ml) for a period of 10 min. A solution of 3-methylbut-1-yn-3-ol (2.54 g, 30.2mmol) in dry di-isopropylamine (10ml) was added dropwise at room temperature; the reaction mixture turned deep orange-brown. The reaction was then heated for 4 hours under gentle reflux under nitrogen. The cooled reaction was filtered through a pad of 'Hyflo-supercel' and water (50ml) added to the filtrate. The crude product was then extracted using diethyl ether (3x50 ml); the combined ethereal extracts were then washed with brine (50ml), dried (MgSO₄), filtered and evaporated to give a brown oil. This was then purified by flash chromatography [fine mesh silica gel; 9:1 dichloromethane-petrol (bp 40-60°C)] to give a brown solid which was recrystallised from cyclohexane and dried *in vacuo* (P₂O₅, 0.30mm Hg, RT, 4 hours).
Yield = 4.70g, (93%)
mp = 43-46°C

Compounds **12-20** have general formula IV, see Table 2.

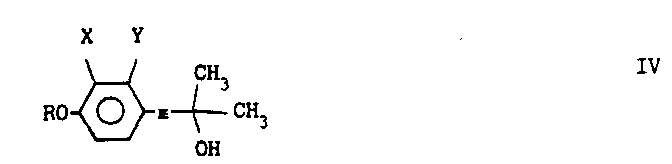

### Compound 13: Preparation of 2-Fluoro-1-(3-hydroxy-3-methylbut-1-ynyl)-4-tridecyloxybenzene

This compound was prepared using a similar method to that described for compound **12**. Quantities: compound **4** (5.18g, 13.9mmol), tetrakis(triphenylphosphine) palladium (O) (1.03g, 0.91mmol), copper (I) iodide (0.68mmol), 3-methylbut-1-yn-3-ol (2.54g, 30.2 mmol) and di-isopropylamine (50ml). The crude product was purified by flash chromatography [fine mesh silica gel; 10% (v/v) ethyl acetate in petrol (bp 40-60°C)] to give a yellow solid which was recrystallised from cyclohexane and dried *in vacuo* (P₂O₅, 0.20mm Hg, RT, 18 hours).
Yield = 3.17g, (58%)
mp = 35-36°C

### Compound 14: Preparation of 2-Fluoro-1-(3-hydroxy-3-methylbut-1-ynyl)-4-tetradecyloxybenzene

This compound was prepared using a similar method to that described for compound **12**. Quantities: compound **5** (5.40g, 13.95 mmol), tetrakis(triphenylphosphine) palladium (O) (1.03g, 0.89mmol), copper (I) iodide (0.14g, 0.74mmol), 3-methylbut-1-yn-3-ol (2.55g, 30.4 mmol) and di-isopropylamine (50ml). The crude product was purified by flash chromatography [fine mesh silica gel; 10% (v/v) ethyl acetate in petrol (bp 40-60°C)] to give a yellow solid which was recrystallised from cyclohexane and dried *in vacuo* (P₂O₅, 0.20mm Hg, RT, 18 hours).
Yield = 2.34g, (43%)
mp. = 44-45°C

### Compound 15: Preparation of 2-Fluoro-1-(3-hydroxy-3-methylbut-1-ynyl)-4-pentadecyloxybenzene

This compound was prepared using a similar method to that described for compound **12**. Quantities: compound 6 (4.22g, 10.5 mmol), tetrakis(triphenylphosphine) palladium (O) (1.0g, 0.87mmol), copper (I) iodide (0.14 g, 0.74mmol), 3-methylbut-1-yn-3-ol (1.90g, 22.6 mmol) and di-isopropylamine (50ml). The crude product was purified by flash chromatography [fine mesh silica gel; 5% (v/v) ethyl acetate in petrol (bp 40-60°C)] to give an orange oil which crystallised on standing. This was recrystallised from cyclohexane and dried *in vacuo* (P₂O₅, 0.20mm Hg, RT, 6 hours).
Yield = 1.57g, (37%)
mp = 40-41°C

### Compound 16: Preparation of 3-Fluoro-4-dodecyloxy-1-(3-hydroxy-3-methylbut-1-ynyl)benzene

This compound was prepared using a similar method to that described for compound **12**. Quantities: compound 7 (5.00g, 13.9 mmol), tetrakis(triphenylphosphine) palladium (O) (1.03g, 0.89mmol), copper (I) iodide (0.12g, 0.63mmol), 3-methylbut-1-yn-3-ol (2.54g, 30.2 mmol) and di-isopropylamine (40ml). The crude orange oil was purified by flash chromatography [fine mesh silica gel; 1:1 dichloromethane-petrol (bp 40-60°C)]; to give an orange solid which was crystallised from cyclohexane and dried *in vacuo* (P₂O₅, 0.30mm Hg, RT, 10 hours).
Yield = 3.58g, (71%)
mp = 30-32°C

### Compound 17: Preparation of 3-Fluoro-1-(3-hydroxy-3-methylbut-1-ynyl)-4-tridecyloxybenzene

This compound was prepared using a similar method to that described for compound **12**. Quantities: compound **8** (5.20g, 13.9 mmol), tetrakis(triphenylphosphine) palladium (O) (1.03g, 0.89mmol), copper (I) iodide (0.14g, 0.74mmol), 3-methylbut-1-yn-3-ol (2.54g, 30.2 mmol) and di-isopropylamine (40ml). The crude product was purified by flash chromatography [fine mesh silica gel; 10% (v/v) ethyl acetate in petrol (bp 40-60°C)]; to give a yellow solid which was recrystallised from cyclohexane and dried *in vacuo* (P₂O₅, 0.20mm Hg, RT, 20 hours).
Yield = 4.63g, (88%)
mp = 46-48°C

### Compound 18: Preparation of 3-Fluoro-1-(3-hydroxy-3-methylbut-1-ynyl)-4-tetradecyloxybenzene

This compound was prepared using a similar method to that described for compound **12**. Quantities: compound **9** (5.40g, 13.9 mmol), tetrakis(triphenylphosphine) palladium (O) (1.05g, 0.91mmol), copper (I iodide (0.13g, 0.68mmol), 3-methylbut-1-yn-3-ol (2.54g, 30.2 mmol) and di-isopropylamine (40ml). The crude product was purified by flash chromatography [fine mesh silica gel; 10% (v/v) ethyl acetate in petrol (bp 40-60°C)]; the white solid recrystallised from cyclohexane and dried *in vacuo* (P₂O₅, 0.40mm Hg, RT, 24 hours).
Yield = 4.16g, (76%)
mp = 45-46°C

### Compound 19: Preparation of 3-Fluoro-1-(3-hydroxy-3-methylbut-1-ynyl)-4-pentadecyloxybenzene

This compound was prepared using a similar method to that described for compound **12**. Quantities: compound **10** (5.58g, 13.9 mmol), tetrakis(triphenylphosphine) palladium (O) (1.02g, 0.88mmol), copper (I) iodide (0.11g, 0.58mmol), 3-methylbut-1-yn-3-ol (2.54g, 30.2 mmol) and di-isopropylamine (50ml). The crude product was purified by flash chromatography [fine mesh silica gel; 5% (v/v) ethyl acetate in petrol (bp 40-60°C)]; the yellow solid was recrystallised from cyclohexane and dried *in vacuo* (P₂O₅, 0.20mm Hg, RT, 72 hours).
Yield = 5.05g, (90%)
mp = 54-55°C

### Compound 20: Preparation of 3-Fluoro-4-hexadecyloxy-1-(3-hydroxy-3-methylbut-1-ynyl)benzene

This compound was prepared using a similar method to that described for compound **12**. Quantities: compound **11** (6.02g, 14.5 mmol), tetrakis(triphenylphosphine) palladium (O) (1.01g, 0.9mmol), copper (I) iodide (0.17g, 0.9mmol), 3-methylbut-1-yn-3-ol (2.46g, 29.2 mmol) and di-isopropylamine (60ml). The crude product was purified by flash chromatography [fine mesh silica gel; 5% (v/v) ethyl acetate in petrol (bp 40-60°C)]; the yellow solid obtained was recrystallised from cyclohexane and dried *in vacuo* (P₂O₅, 0.20mm Hg, RT, 5 hours).
Yield = 4.68g, (77%)
mp = 49-50°C

### Compound 21: 4-Dodecyloxy-1-ethynyl-2-fluorobenzene

Compound **12** (3.85g, 10.6mmol), potassium hydroxide (0.62g, 11.1mmol) and toluene (100ml) were stirred and heated under reflux under nitrogen for 2 hours. The acetone and toluene azeotrope was removed periodically via a Dean and Stark receiver and replaced with an equal volume of toluene. The cooled reaction mixture was poured onto water (100ml) and the organic phase separated. The aqueous phase was then washed with diethyl ether (3x50ml) and recombined with the organic layer before being washed with brine (50ml), dried (MgSO₄), filtered and evaporated to give an orange oil. This was purified by flash chromatography [fine mesh silica gel; petrol (bp 40-60°C)] the yellow solid obtained was recrystallised (cyclohexane) and dried *in vacuo* (P₂O₅, 0.30mm Hg, RT, 5 hours).
Yield = 2.28g, (71%)
mp = 35-36°C

Compounds 21-29 have general formula V, see Table 3.

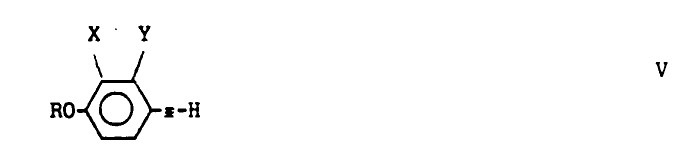

### Compound 22: 1-Ethynyl-2-fluoro-4-tridecyloxybenzene

This compound was prepared using a similar method to that described for compound **21**. Quantities: compound **13** (3.08g, 8.19mmol) potassium hydroxide (0.53g, 9.45mmol and toluene (80ml). The crude product was purified by flash chromatography [fine mesh silica gel; 9:1 dichloromethane-petrol (bp 40-60°C)]; the brown solid obtained was recrystallised from cyclohexane and dried *in vacuo* (P₂O₅, 0.10mm Hg, 40°C, 7 hours).
Yield = 1.89g, (72%)
mp = 40-41°C

### Compound 23: 1-Ethynyl-2-fluoro-4-tetradecyloxybenzene

This compound was prepared using a similar method to that described for compound **21**. Quantities: compound **14** (2.35g, 6.02mmol) potassium hydroxide (0.39g, 6.95mmol and toluene (70ml). The crude product was purified by flash chromatography [fine mesh silica gel; dichloromethane]; the yellow solid obtained was recrystallised from cyclohexane and dried *in vacuo* (P₂O₅, 0.10mm Hg, 40°C, 3 hours).
Yield = 1.28g, (64%)
mp = 41-42°C

### Compound 24: 1-Ethynyl-2-fluoro-4-pentadecyloxybenzene

This compound was prepared using a similar method to that described for compound **21**. Quantities: compound **15** (1.53g, 3.8mmol) potassium hydroxide (0.26g, 4.6mmol) and toluene (100ml). The crude product was purified by flash chromatography [fine mesh silica gel; dichloromethane]; the yellow solid obtained was recrystallised from cyclohexane and dried *in vacuo* (P₂O₅, 0.30mm Hg, RT, 18 hours).
Yield = 1.17g, (88%)
mp = 41-42°C

### Compound 25: 1-Ethynyl-3-fluoro-4-dodecyloxybenzene

This compound was prepared using a similar method to that described for compound **21**. Quantities: compound **16** (3.49g, 9.64mmol) potassium hydroxide (0.62g, 11.07mmol and toluene (100ml). The crude product was purified by flash chromatography [fine mesh silica gel; 9:1 dichloromethane-petrol (bp 40-60°C)]; the yellow solid obtained was dried *in vacuo* (P₂O₅, 0.40mm Hg, RT, 6 hours).
Yield = 2.65g, (90%)
mp - not recorded

### Compound 26: 1-Ethynyl-3-fluoro-4-tridecyloxybenzene

This compound was prepared using a similar method to that described for compound **21** Quantities: compound **17** (4.56g, 12.2mmol) potassium hydroxide (0.76g, 13.5mmol and toluene (100ml). The crude product was purified by flash chromatography [fine mesh silica gel; 9:1 dichloromethane-petrol (bp 40-60°C]; the yellow solid obtained was dried *in vacuo* (P₂O₅, 0.40mm Hg, RT, 4 hours).
Yield = 3.54g, (91%)
mp = 39-41°C

### Compound 27: 1-Ethynyl-3-fluoro-4-tetradecyloxybenzene

This compound was prepared using a similar method to that described for compound **21**. Quantities: compound **18** (4.06g, 10.4mmol) potassium hydroxide (0.62g, 11.1mmol and toluene (100ml). The crude product was purified by flash chromatography [fine mesh silica gel; 9:1 dichloromethane-petrol (bp 40-60°C)]; the yellow solid obtained was dried *in vacuo* (P₂O₅, 0.35mm Hg, RT, 6 hours).
Yield = 3.08g, (89%)
mp = 34-35°C

### Compound 28: 1-Ethynyl-3-fluoro-4-pentadecyloxybenzene

This compound was prepared using a similar method to that described for compound **21**. Quantities: compound **19** (4.99g, 12.35mmol) potassium hydroxide (0.73g, 13.01mmol and toluene (100ml). The crude product was purified by flash chromatography [fine mesh silica gel; 9:1 dichloromethane-petrol (bp 40-60°C)]; the orange solid obtained was recrystallised from cyclohexane and dried *in vacuo* (P₂O₅, 0.20mm Hg, 40°C, 5 hours).
Yield = 2.69g, (68%)
mp = 45-47°C

### Compound 29: 1-Ethynyl-3-fluoro-4-hexadecyloxybenzene

This compound was prepared using a similar method to that described for compound **21**. Quantities: compound **20** (4.61g, 8.8mmol) potassium hydroxide (0.52g, 9.2mmol) and toluene (120ml). The crude product was purified by flash chromatography [fine mesh silica gel; dichloromethane]; the yellow solid obtained was dried *in vacuo* (P₂O₅, 0.30mm Hg, RT, 18 hours).
Yield = 2.89g, (91%)
mp = 37-39°C

### Compound 30: 3-(2-Fluoro-4-dodecyloxyphenyl)propiolic acid

Butyllithium (2.8ml, 2.5M in hexanes) was added dropwise to a stirred, cooled (-78°C) solution of compound 21 (2.10g, 6.91mmol) in dry thf (100ml) under nitrogen. The resulting solution was kept at -78°C for a further 1.5 hours before being poured onto a stirred slurry of crushed 'Cardice' and dry thf (100ml) and allowed to warm to room temperature overnight. The solution was acidified with concentrated hydrochloric acid and water (100ml) added. The product was extracted with diethyl ether (3x50ml), The combined ethereal extracts were then washed with brine (50ml), dried (MgSO₄), filtered and evaporated to give a yellow solid. The product was purified by flash chromatography [fine mesh silica gel; dichloromethane (initially) and 9:1 dichloromethane-methanol (finally)]. Two fractions were obtained and recrystallised from cyclohexane and dried *in vacuo* (P₂O₅, 0.30mm Hg, RT, 10 hours), the first proving to be unreacted compound **21**.
Yield = 0.87g, (36%)
mp - not recorded

Compounds 30-38 have general formula VI, see Table 4.

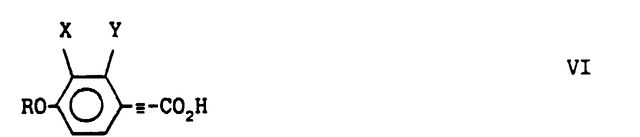

### Compound 31: 3-(2-Fluoro-4-tridecyloxyphenyl)propiolic acid

Butyllithium (2.4ml, 2.5M in hexanes) was added dropwise to a stirred, cooled (-10°C) solution of compound 22 (1.80g, 5.66mmol) in dry thf (40ml) under nitrogen. The reaction was stirred for a further 10 min at -10°C before being poured onto a stirred slurry of crushed 'Cardice' and dry thf (20ml) with stirring and allowed to warm to room temperature. The solution was acidified with concentrated hydrochloric acid and then diluted with water (100ml). The organic phase was then separated and the aqueous phase washed with diethyl ether (2x50ml), The combined organic phases were then washed with brine (50ml), dried (MgSO₄), filtered and evaporated to give a brown liquid. The product was purified by flash chromatography [fine mesh silica gel; dichloromethane (initially) and 9:1 dichloromethane-methanol (finally)]; to give a brown solid which was recrystallised from cyclohexane and dried *in vacuo* (P₂O₅, 0.20mm Hg, 40°C, 5 hours).
Yield = 0.84g, (41%)
mp = 98-99°C

### Compound 32: 3-(2-Fluoro-4-tetradecyloxyphenyl)propiolic acid

This compound was prepared using a similar method to that described for compound **31**. Quantities: compound **23** (1.21g, 3.6mmol), butyllithium (1.5ml, 2.5M in hexanes) and dry thf (35ml). The crude product was purified by flash chromatography [fine mesh silica gel; dichloromethane (initially) and 9:1 dichloromethane-methanol (finally)]; to give a brown solid which was recrystallised (ethanol) and dried *in vacuo* (P₂O₅, 0.20mm Hg, 40°C, 5 hours).
Yield = 0.97g, (71%)
mp = 90-109(dec)°C

### Compound 33: 3-(2-Fluoro-4-pentadecyloxyphenyl)propiolic acid

This compound was prepared using a similar method to that described for compound **31**. Quantities: compound **24** (1.16g, 3.4mmol), butyllithium (1.4ml, 2.5M in hexanes) and dry thf (35ml). The crude product was purified by flash chromatography [fine mesh silica gel; dichloromethane (initially) and 9:1 dichloromethane-methanol (finally)]; to give a dark green solid which was recrystallised (cyclohexane) and dried *in vacuo* (P₂O₅, 0.30mm Hg, 35°C, 5 hours).
Yield = 0.77g, (58%)
mp = 100-102°C

### Compound 34: 3-(3-Fluoro-4-dodecyloxyphenyl)propiolic acid

This compound was prepared using a similar method to that described for compound **31**. Quantities: compound **25** (2.48g, 8.16mmol), butyllithium (3.3ml, 2.5M in hexanes) and dry thf (40ml). The pale yellow solid obtained from flash chromatography was recrystallised (cyclohexane) and dried *in vacuo* (P₂O₅, 0.10mm Hg, 40°C, 4 hours).
Yield = 1.70g, (60%)
mp = 89-93°C

### Compound 35: 3-(3-Fluoro-4-tridecyloxyphenyl)propiolic acid

This compound was prepared using a similar method to that described for compound **31**. Quantities: compound **26** (1.71g, 5.4mmol), butyllithium (2.2ml, 2.5M in hexanes) and dry thf (35ml). The brown solid obtained after flash chromatography was recrystallised (cyclohexane) and dried *in vacuo* (P₂O₅, 0.10mm Hg, 40°C, 18 hours).
Yield = 1.12g, (57%)
mp = 91-94°C

### Compound 36: 3-(3-Fluoro-4-tetradecyloxyphenyl)propiolic acid

This compound was prepared using a similar method to that described for compound **31**. Quantities: compound **27** (3.03g, 9.1mmol), butyllithium (3.8ml, 2.5M in hexanes) and dry thf (35ml). The yellow solid obtained after flash chromatography was recrystallised (cyclohexane-ethyl acetate), washed with petrol (bp 40-60°C) (1ml) and dried *in vacuo* (P₂O₅, 0.10mm Hg, 45°C, 5 hours).
Yield = 2.47g, (72%)
mp = 91-93°C

### Compound 37: 3-(3-Fluoro-4-pentadecyloxyphenyl)propiolic acid

This compound was prepared using a similar method to that described for compound **31**. Quantities: compound **28** (2.62g, 7.57mmol), butyllithium (3.2ml, 2.5M in hexanes) and dry thf (50ml). The light yellow solid obtained after flash chromatography was recrystallised (cyclohexane) and dried *in vacuo* (P₂O₅, 0.10mm Hg, 40°C, 5 hours).
Yield = 1.67g, (56%)
mp = 95-97°C

### Compound 38: 3-(3-Fluoro-4-hexadecyloxyphenyl)propiolic acid

This compound was prepared using a similar method to that described for compound **31**. Quantities: compound **29** (3.58g, 9.9mmol), butyllithium (4.00ml, 2.5M in hexanes) and dry thf (50ml). The crude product was purified by flash chromatography [fine mesh silica gel; dichloromethane (initially) and 9:1 dichloromethane-methanol (finally)]; the yellow solid obtained was recrystallised (cyclohexane) and dried *in vacuo* (P₂O₅, 0.30mm Hg, 30°C, 5 hours).
Yield = 2.53g, (63%)
mp = 93-96°C

### Compound 40: 4'-Hydroxybiphenyl-4-carboxylic acid

A mixture of concentrated sulphuric acid (115ml) and water (115ml) was added dropwise to a stirred suspension of 4-cyano-4'-hydroxybiphenyl **39** (25.62g, 131.4mmol) in glacial acetic acid (400ml). The mixture was heated under reflux for 48 hours, the cooled reaction mixture was then poured into water (600ml) with stirring and the white precipitate filtered off. The aqueous filtrate was then washed with diethyl ether (4x70ml); the combined extracts were then washed with water (50ml), dried (MgSO₄) filtered and evaporated to give a white solid. both crops of product were combined, dried thoroughly and recrystallised (glacial acetic acid) and dried *in vacuo* (P₂O₅, 0.30mm Hg, 50°C, 5 hours).
Yield = 23.49g, (84%)
mp - not recorded

### Compound 41: 4'-Methoxycarbonyloxybiphenyl-4-carboxylic acid

Compound **40** (15.02g, 70.2mmol) was added slowly to a vigorously stirred solution of sodium hydroxide (8.15g, 203.8mmol) in water (300ml) at -4°C. Methyl chloroformate (10.82g, 114.5mmol) was added dropwise and the temperature maintained at 0°C. The resulting white slurry was then stirred under these conditions for a further 4 hours. The pH was then adjusted to 5 using concentrated hydrochloric acid solution (1:1, conc HCl-water) and the voluminous white precipitate filtered off and washed with water. the white solid was dried and recrystallised (glacial acetic acid) and dried *in vacuo* (P₂O₅, 0.20mm Hg, 40°C, 4 hours).
Yield = 14.83g, (78%)
mp = 256-260°C

### Compound 42: (R)-1-Methylheptyl 4'-methoxycarbonyloxybiphenyl-4-carboxylate

Triphenylphosphine (6,80g, 25.9mmol) and (S)-octan-2-ol (5.03g, 38.6mmol) in dry thf (35ml) were added dropwise to a stirred mixture of compound **41** (7.03g, 25.8mmol) and diethyl azodicarboxylate (4.50g, 25.9mmol) in dry thf (40ml) under nitrogen. The reaction was stirred for a further 24 hours at room temperature. The white precipitate was removed by filtration through a pad of 'Hyflo-supercel', the filtrate was then washed with brine (50ml), dried (MgSO₄), filtered and evaporated to give a white solid. This was purified by flash chromatography [fine mesh silica gel; 5% (v/v) ethyl acetate in petrol (bp 40-60°C)] to give a colourless oil which was dried *in vacuo* (P₂O₅, 0.20mm Hg, RT, 5 hours).
Yield = 8.06g, (81%)

### Compound 43: (S)-1-Methylheptyl 4'-methoxycarbonyloxybiphenyl-4-carboxylate

This compound was prepared using a similar method to that described for compound **42**. Quantities: triphenylphosphine (6.74g, 25.7mmol), (R)-octan-2-ol (5.03g, 38.7mmol), compound 41 (7.00g, 25.7mmol), diethyl azodicarboxylate (4.50g, 25.9mmol) and dry thf (110ml). The crude product was purified by flash chromatography [fine mesh silica gel; 5% (v/v) ethyl acetate in petrol (bp 40-60°C)]; to give a colourless liquid which was dried *in vacuo* (P₂O₅, 0.20mm Hg, RT, 5 hours).
Yield = 7.98g, (81%)

### Compound 44: (R)-1-Methylheptyl 4'-hydroxybiphenyl-4-carboxylate

A solution of compound **42** (8.06g, 20.9mmol) in ethanol (30ml) was added dropwise to a stirred mixture of ammonia (105ml, 35% solution) and ethanol (180ml) at room temperature. TLC analysis showed complete reaction after a period of 30 min. The reaction was then poured into water (300ml) and cooled in 'Cardice', the precipitated product was then filtered off, dried and recrystallised (cyclohexane-ethyl acetate, 4:1). the colourless crystals were then dried *in vacuo* (P₂O₅, 0.10mm Hg, 40°C, 5 hours).
Yield = 5.52g, (81%)
mp = 84-87°C

### Compound 45: (S)-1-Methylheptyl 4'-hydroxybiphenyl-4-carboxylate

This compound was prepared using a similar method to that described for compound **44**. Quantities: compound **43** (7.51g, 19.6mmol), ethanol (210ml) and ammonia (105ml, 35% solution).
Yield = 4.89g, (77%)
mp = 87-89°C

### Compound 46: (R)-1-Methylheptyl 4'-[(2-fluoro-4-dodecyloxyphenyl)propioloyloxy)biphenyl-4-carboxylate

Dicyclohexylcarbodiimide (0.31g, 1.5mmol) was added to a stirred mixture of compound **30** (0.42g, 1.2mmol), compound **44** (0.39g, 1.2mmol), 4-N,N-dimethylaminopyridine (0.05g, 0.4mmol) in dry dichloromethane (10ml) at room temperature and then stirred for a further 18 hours. The reaction was diluted with dichloromethane (50ml) and the precipitate was removed by filtration through a pad of `Hyflo-supercel' and the filtrate washed successively with water (50ml), 5% (v/v) acetic acid solution (2x50ml), water (50ml) before being dried (MgSO₄), filtered and evaporated to give an orange solid. This was purified twice by flash chromatography, first [fine mesh silica gel; 5% (v/v) ethyl acetate in petrol (bp 40-60°C)] and then [fine mesh silica gel; 9:1 dichloromethane-petrol (bp 40-60°C)] to give a colourless solid which was recrystallised (cyclohexane, twice) and dried *in vacuo* (P₂O₅, 0.20mm Hg, 50°C, 7 hours).
Yield = 0.17g, (22%)
Phase Transition Temperatures/°C (on cooling)
I 87.1 S_{A} 86.8 S_{C}* 49.8 K

Compounds 46-53 have general formula VII, see Table 5.

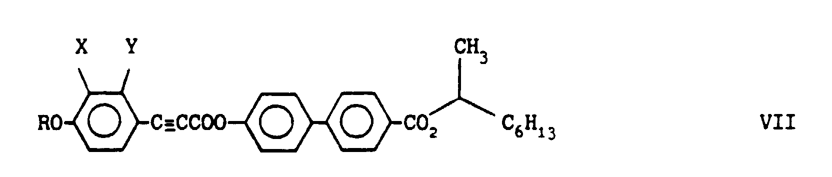

### Compound 47: (S)-1-Methylheptyl 4'-[(2-fluoro-4-dodecyloxyphenyl)propioloyloxy)biphenyl-4-carboxylate

This compound was prepared using a similar method to that described for compound **46**. Quantities: dicyclohexylcarbodiimide (0.31g, 1.5mmol), compound **30** (0.40g, 1.2mmol), compound **45** (0.33g, 1.0mmol), 4-N,N-dimethylaminopyridine (0.04g, 0.3mmol) and dry dichloromethane (16ml). The crude product was purified twice by flash chromatography, first [fine mesh silica gel; 5% (v/v) ethyl acetate in petrol (bp 40-60°C)] and then [fine mesh silica gel; 9:1 dichloromethane-petrol (bp 40-60°C)]; to give a white solid which was recrystallised (cyclohexane, three times) and dried *in vacuo* (P₂O₅, 0.30mm Hg, 40°C, 5 hours).
Yield = 0.24g, (36%)
I 87.1 S_{A} 86.5 S_{C}* 57.6 K

### Compound 48: (R)-1-Methylheptyl 4'-[(3-fluoro-4-dodecyloxyphenyl)propioloyloxy)biphenyl-4-carboxylate

This compound was prepared using a similar method to that described for compound **46**. Quantities: dicyclohexylcarbodiimide (0.37g, 1.8mmol), compound **34** (0.57g, 1.6mmol), compound **44** (0.51g, 1.6mmol). 4-N,N-dimethylaminopyridine (0.06g, 0.5mmol) and dry dichloromethane (10ml). The crude product was purified twice by flash chromatography, first [fine mesh silica gel; 5% (v/v) ethyl acetate in petrol (bp 40-60°C)] and then [fine mesh silica gel; 9:1 dichloromethane-petrol (bp 40-60°C)]; to give a colourless solid which was recrystallised (cyclohexane, twice) and dried *in vacuo* (P₂O₅, 0.20mm Hg, 40°C, 5 hours).
Yield = 0.31g, (30%)
I 75.1 S_{C}* 74.0 K

### Compound 49: (S)-1-Methylheptyl 4'-[(3-fluoro-4-dodecyloxyphenyl)propioloyloxy)biphenyl-4-carboxylate

This compound was prepared using a similar method to that described for compound **46**. Quantities: dicyclohexylcarbodiimide (0.37g, 1.8mmol), compound **34** (0.54g, 1.6mmol), compound **45** (0.50g, 1.6mmol), 4-N,N-dimethylaminopyridine (0.04g, 0.3mmol) and dry dichloromethane (10ml). The crude product was purified twice by flash chromatography, first [fine mesh silica gel; 5% (v/v) ethyl acetate in petrol (bp 40-60°C)] and then [fine mesh silica gel; 9:1 dichloromethane-petrol (bp 40-60°C)]; to give a colourless solid which was recrystallised (cyclohexane, three times) and dried *in vacuo* (P₂O₅, 0.10mm Hg, 40°C, 72 hours).
Yield = 0.47g, (47%)
I 75.5 S_{C}* 58.2 K

### Compound 50: (R)-1-Methylheptyl 4'-[(3-fluoro-4-tridecyloxyphenyl)propioloyloxy)biphenyl-4-carboxylate

This compound was prepared using a similar method to that described for compound **46**. Quantities: dicyclohexylcarbodiimide (0.54g, 2.62mmol), compound 35 (0.56g, 1.6mmol), compound **44** (0.51g, 1.6mmol), 4-N,N-dimethylaminopyridine (0.05g, 0.4mmol) and dry dichloromethane (10ml). The crude product was purified twice by flash chromatography, first [fine mesh silica gel; 5% (v/v) ethyl acetate in petrol (bp 40-60°C)] and then [fine mesh silica gel; 9:1 dichloromethane-petrol (bp 40-60°C)]; to give a colourless solid which was recrystallised (cyclohexane) and dried *in vacuo* (P₂O₅, 0.10mm Hg, 50°C, 6 hours).
Yield = 0.16g, (15%)
I 74.6 S_{C}* 66.5 K

### Compound 51: (S)-1-Methylheptyl 4'-[(3-fluoro-4-tridecyloxyphenyl)propioloyloxy)biphenyl-4-carboxylate

This compound was prepared using a similar method to that described for compound **46**. Quantities: dicyclohexylcarbodiimide (0.40g, 1.9mmol), compound **35** (0.58g, 1.6mmol), compound **45** (0.51g, 1.6mmol), 4-N,N-dimethylaminopyridine (0.05g, 0.4mmol) and dry dichloromethane (10ml). The crude product was purified twice by flash chromatography, first [fine mesh silica gel; 5% (v/v) ethyl acetate in petrol (bp 40-60°C)] and then [fine mesh silica gel; 9:1 dichloromethane-petrol (bp 40-60°C)]; to give a colourless solid which was recrystallised (cyclohexane, twice) and dried *in vacuo* (P₂O₅, 0.10mm Hg, 40°C, 8 hours).
Yield = 0.13g, (12%)
I 74.2 S_{C}* 63.5 K

### Compound 52: (R)-1-Methylheptyl 4'-[(3-fluoro-4-tetradecyloxyphenyl)propioloyloxy)biphenyl-4-carboxylate

This compound was prepared using a similar method to that described for compound **46**. Quantities: dicyclohexylcarbodiimide (0.35g, 1.7mmol), compound **36** (0.59g, 1.6mmol), compound **44** (0.50g, 1.5mmol), 4-N,N-dimethylaminopyridine (0.04g, 0.3mmol) and dry dichloromethane (10ml). The crude product was purified twice by flash chromatography, first [fine mesh silica gel; 5% (v/v) ethyl acetate in petrol (bp 40-60°C)] and then [fine mesh silica gel; 9:1 dichloromethane-petrol (bp 40-60°C)]; to give a colourless solid which was recrystallised (cyclohexane, three times) and dried *in vacuo* (P₂O₅, 0.40mm Hg, 40°C, 6 hours).
Yield = 0.15g, (14%)
I 75.4 S_{C}* 49.6 K

### Compound 53: (S)-1-Methylheptyl 4'-[(3-fluoro-4-tetradecyloxyphenyl)propioloyloxy)biphenyl-4-carboxylate

This compound was prepared using a similar method to that described for compound **46**. Quantities: dicyclohexylcarbodiimide (0.37g, 1.8mmol), compound **36** (0.58g, 1.5mmol), compound **45** (0.50g, 1.5mmol). 4-N,N-dimethylaminopyridine (0.03g, 0.25mmol) and dry dichloromethane (10ml). The crude product was purified twice by flash chromatography, first [fine mesh silica gel; 5% (v/v) ethyl acetate in petrol (bp 40-60°C)] and then [fine mesh silica gel; 9:1 dichloromethane-petrol (bp 40-60°C)]; to give a colourless solid which was recrystallised (cyclohexane) and dried *in vacuo* (P₂O₅, 0.30mm Hg, 40°C, 17 hours).
Yield = 0.18g, (17%)
I 75.5 S_{C}* 62.7 K

Compounds of formula I may be mixed with a wide range of hosts, for example smectic hosts to form a useful liquid crystal composition. Such compositions can have a range of Ps values. Compounds of formula I may be mixed with one or more of the types of hosts VIII - XIII. These different types of hosts may be mixed together to which the compound of general formula I may also be added.

### Typical hosts include:

The compounds described in PCT/GB86/00040, eg of formula VIII 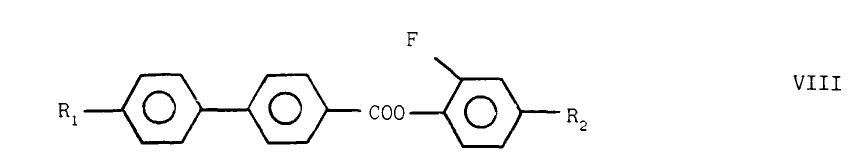 where R₁ and R₂ are independently C₃-C₁₂ alkyl or alkoxy.

The fluoro-terphenyls described in EPA 84304894.3 and GBA 8725928, eg of formula IX 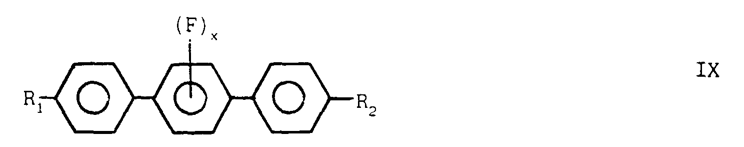 where R₁ and R₂ are independently C₁-C₁₂ alkyl or alkoxy, x is 1 and F may be on any of the available substitution positions on the phenyl ring specified.

The difluoro-terphenyls described in GBA 8905422.5, eg of formula X 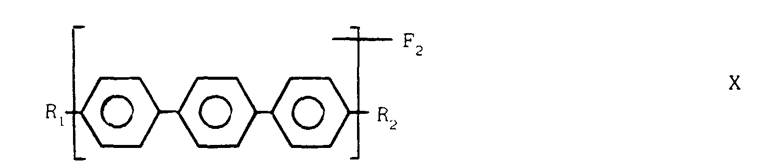 where R₁ and R₂ are independently C₃-C₁₂ alkyl or alkoxy.

The phenyl-pyrimidines described in WO 86/00087, eg of formula XI 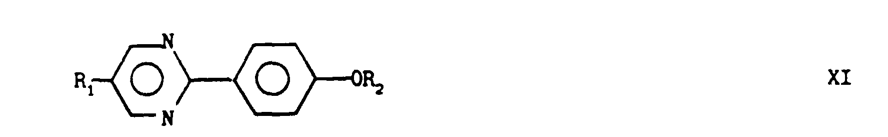 including those compounds where R₁ is C₃-C₁₂ alkyl and R₂ is given by the general formula (CH₂)ₙ-CHXCH₂CH₃, where n is 1 to 5 and X is CN or Cl.

The compounds described by R. Eidenschink *et. al*. in Cyclohexanederivative mit Getilteneten Smektischen Phasen at the 16^{th} Freiberg Liquid Crystal Conference, Freiberg, Germany, p8. Available from E. Merck Ltd., Germany, eg of formula XII. 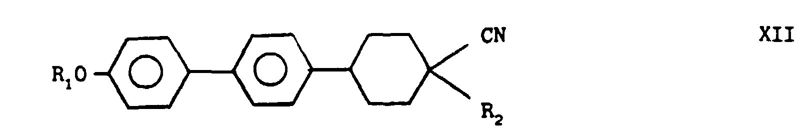 including those compounds where R₁ and R₂ are independently C₁-C₁₅ alkyl or in the case of R₂ alkoxy.

The difluoro-phenyl pyrimidines described at the 2^{nd} International Symposium on Ferroelectric Liquid Crystals, Göteborg, Sweden, June 1989 by Reiffenrath *et. al*., eg of formula XIII 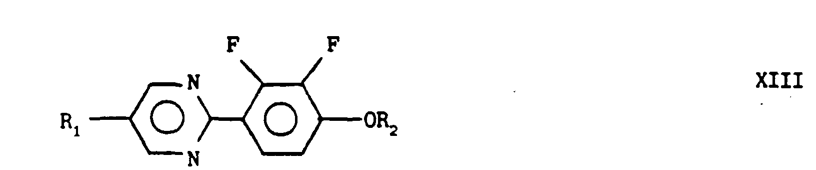 including those compounds where R₁ and R₂ are independently C₃-C₉ alkyl in the case of R₁ alkoxy.

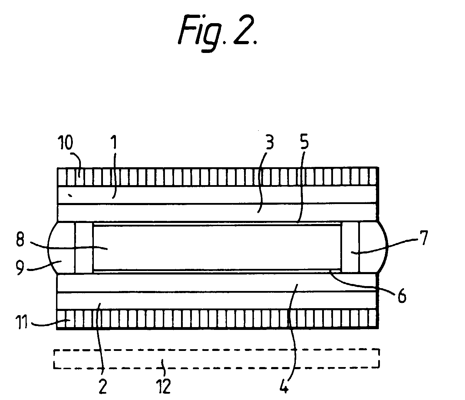

An example of the use of a compound of Formula I in a liquid crystal material and device embodying the present invention will now be described with reference to Figure 2.

The liquid crystal device consists of two transparent plates, 1 and 2, in this case made from glass. These plates are coated on their internal face with transparent conducting electrodes 3 and 4. An alignment layer is introduced onto the internal faces of the cell so that a planar orientation of the molecules making up the liquid crystalline material will be approximately parallel to the glass plates 1 and 2. This is done by coating the glass plates 1,2 complete with conducting electrodes 3,4 with layers of film 5 and 6 of a suitable polymer, eg polyimide. The electrodes 3,4 may be formed into row and column electrodes so that the intersections between each column and row form an x, y matrix of addressable elements or pixels. Prior to the construction of the cell the films 5,6 are rubbed with a soft tissue in a given direction, the rubbing directions being arranged parallel upon construction of the cell. A spacer 7 eg of polymethyl methacrylate separates the glass plates 1 and 2 to a suitable distance eg 2 microns. Liquid crystal material 8 is introduced between glass plates 1, 2 by filling the space in between them. The spacer 7 is sealed with an adhesive 9 in a vacuum using an existing technique. Polarisers 10, 11 are arranged in front of and behind the cell.

The device may operate in a transmissive or reflective mode. In the former, light passing through the device, eg from a tungsten bulb, is selectively transmitted or blocked to form the desired display. In the reflective mode a mirror (12) is placed behind the second polariser 11 to reflect ambient light back through the cell and two polarisers. By making the mirror partly reflecting the device may be operated both in a transmissive and reflective mode.

Tables 8-11 give values for the spontaneous polarisation (Ps/nC/cm²) over a range of temperatures (°C) for a number of the compounds described by general formula I. The compounds of formula I may be added to host materials. Table 12 gives values for the spontaneous polarisation (Ps/nC/cm²) over a range of temperatures (°C) when it is mixed 10% by weight with host material H1. H1 is a 1:1:1 mixture of the following:
R₁ = C₈H₁₇, R₂ = C₅H₁₁
R₁ = OC₈H₁₇, R₂ = C₅H₁₁
R₁ = OC₈H₁₇, R₂ = C₇H₁₅

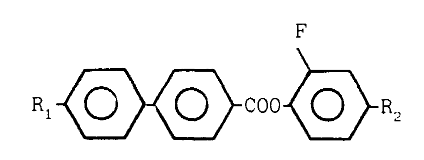

The host is a commercially available host and is widely used in ferroelectric liquid crystal mixtures.

The Ps was measured in a 6µm polyimide (PI) parallel cell using a Diamant bridge and applying a sine wave at 30Hz.

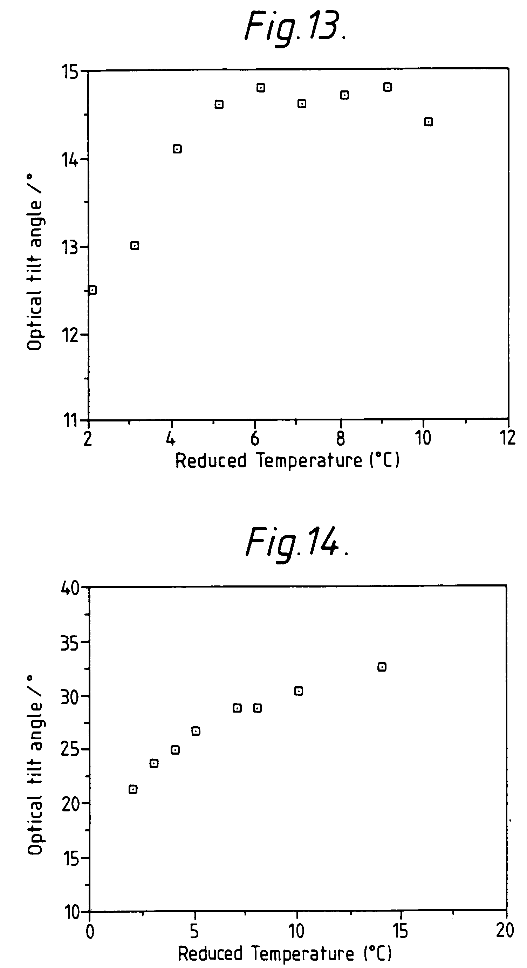

The optical tilt angle measurements detailed in Figures 13 and 14 show the dependence of the optical tilt angle of the S_{c}* phase with reduced temperature and the studies were performed in 3.6µm thick polyimide coated cells.

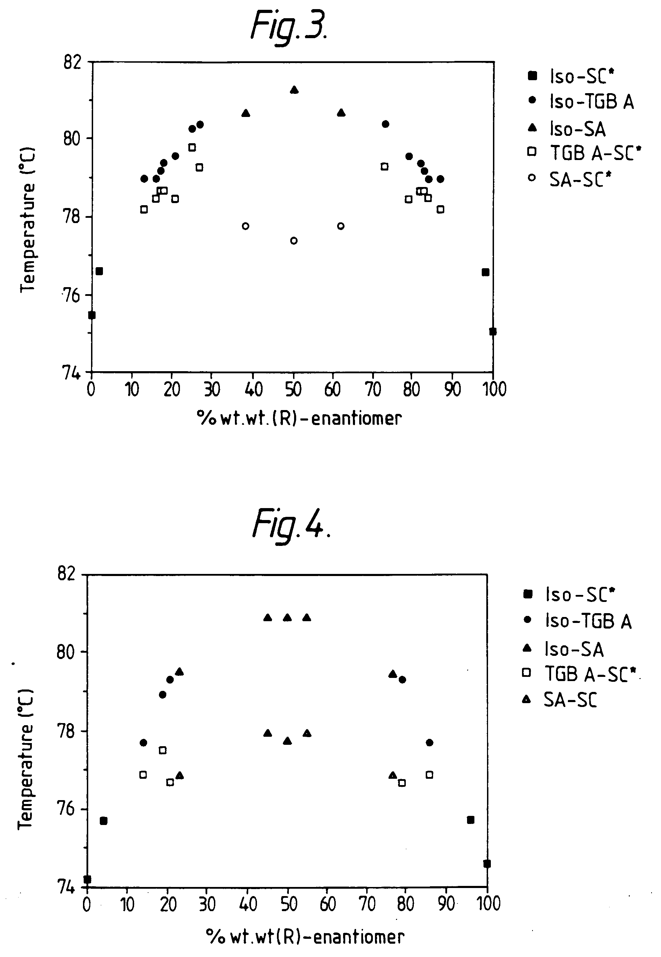

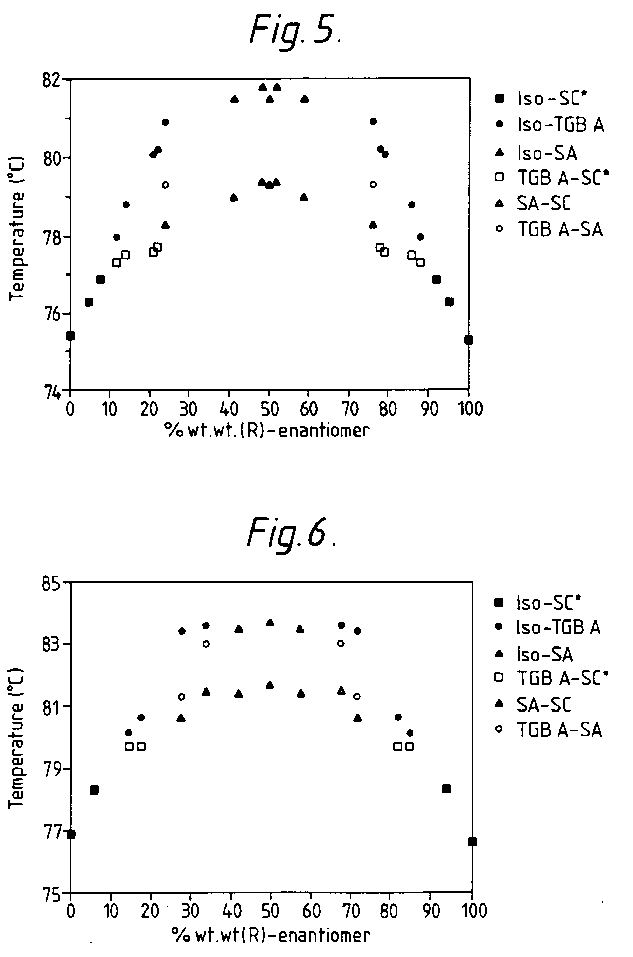

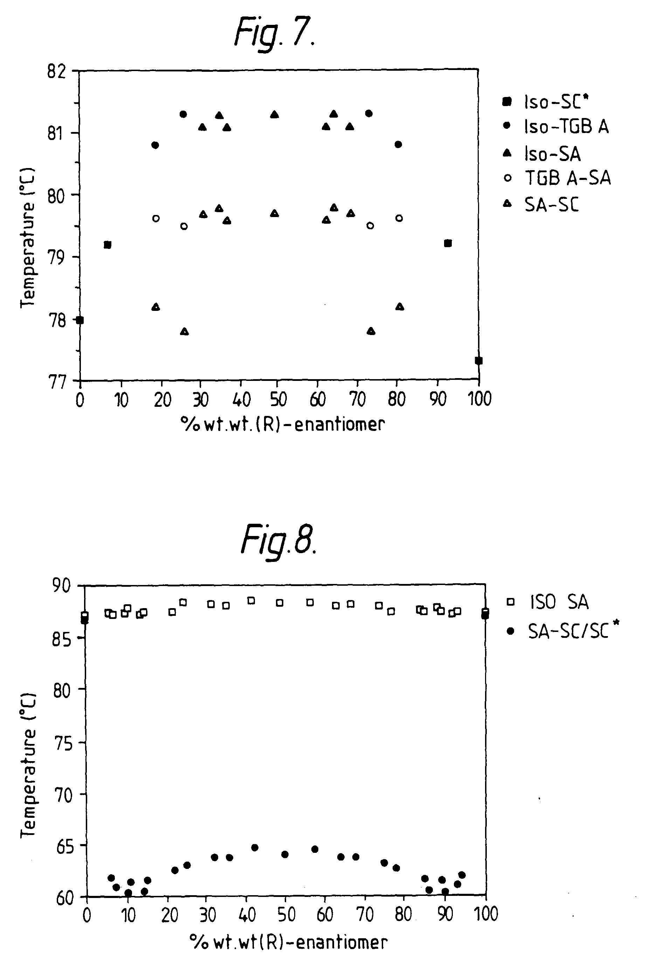

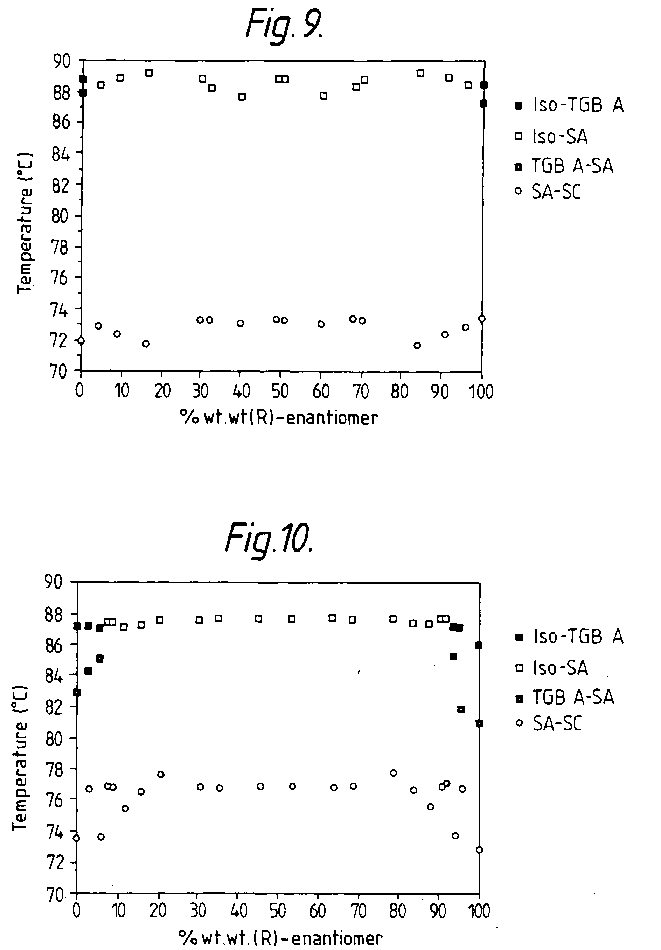

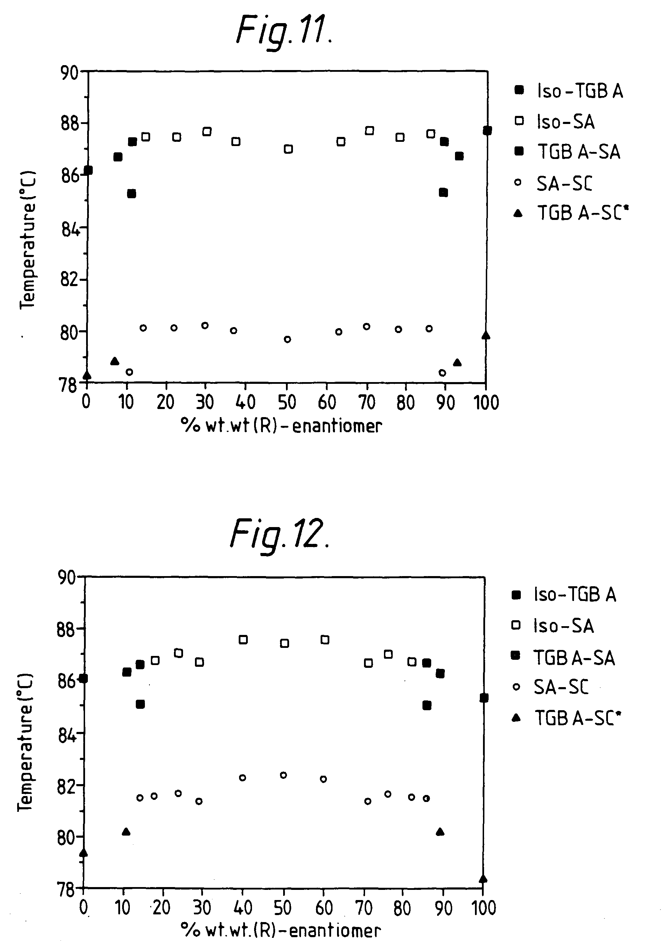

Figures 3-12 are phase diagrams for compounds 48/49; 50/51; 52/53; 60/61; 64/65; 46/47; 54/55; 56/57; 58/59; 62/63.

**Table 1**

| Compound | R | X | Y |
|---|---|---|---|
| 3 | C₁₂H₂₅ | H | F |
| 4 | C₁₃H₂₇ | H | F |
| 5 | C₁₄H₂₉ | H | F |
| 6 | C₁₅H₃₁ | H | F |
| 7 | C₁₂H₂₅ | F | H |
| 8 | C₁₃H₂₇ | F | H |
| 9 | C₁₄H₂₉ | F | H |
| 10 | C₁₅H₃₁ | F | H |
| 11 | C₁₆H₃₃ | F | H |

**Table 2**

| Compound | R | X | Y |
|---|---|---|---|
| 12 | C₁₂H₂₅ | H | F |
| 13 | C₁₃H₂₇ | H | F |
| 14 | C₁₄H₂₉ | H | F |
| 15 | C₁₅H₃₁ | H | F |
| 16 | C₁₂H₂₅ | F | H |
| 17 | C₁₃H₂₇ | F | H |
| 18 | C₁₄H₂₉ | F | H |
| 19 | C₁₅H₃₁ | F | H |
| 20 | C₁₆H₃₃ | F | H |

**Table 3**

| Compound | R | X | Y |
|---|---|---|---|
| 21 | C₁₂H₂₅ | H | F |
| 22 | C₁₃H₂₇ | H | F |
| 23 | C₁₄H₂₉ | H | F |
| 24 | C₁₅H₃₁ | H | F |
| 25 | C₁₂H₂₅ | F | H |
| 26 | C₁₃H₂₇ | F | H |
| 27 | C₁₄H₂₉ | F | H |
| 28 | C₁₅H₃₁ | F | H |
| 29 | C₁₆H₃₃ | F | H |

**Table 4**

| Compound | R | X | Y |
|---|---|---|---|
| 30 | C₁₂H₂₅ | H | F |
| 31 | C₁₃H₂₇ | H | F |
| 32 | C₁₄H₂₉ | H | F |
| 33 | C₁₅H₃₁ | H | F |
| 34 | C₁₂H₂₅ | F | H |
| 35 | C₁₃H₂₇ | F | H |
| 36 | C₁₄H₂₉ | F | H |
| 37 | C₁₅H₃₁ | F | H |
| 38 | C₁₆H₃₃ | F | H |

**Table 5**

| Compound | R | X | Y |
|---|---|---|---|
| 46 (R)* | C₁₂H₂₅ | H | F |
| 47 (S) | C₁₂H₂₅ | H | F |
| 48 (R) | C₁₂H₂₅ | F | H |
| 49 (S) | C₁₂H₂₅ | F | H |
| 50 (R) | C₁₃H₂₇ | F | H |
| 51 (S) | C₁₃H₂₇ | F | H |
| 52 (R) | C₁₄H₂₉ | F | H |
| 53 (S) | C₁₄H₂₉ | F | H |

| | | | |
|---|---|---|---|
| * optical isomer | | | |

In a manner similar to that for compounds 46-53, compounds 54-65 were also synthesised. Compounds 54-65 have the same general formula as those compounds in Table 5 and are presented in Table 6.

**Table 6**

| Compound | R | X | Y |
|---|---|---|---|
| 54 (R)* | C₁₃H₂₇ | H | F |
| 55 (S) | C₁₃H₂₇ | H | F |
| 56 (R) | C₁₄H₂₉ | H | F |
| 57 (S) | C₁₄H₂₉ | H | F |
| 58 (R) | C₁₅H₃₁ | H | F |
| 59 (S) | C₁₅H₃₁ | H | F |
| 60 (R) | C₁₅H₃₁ | F | H |
| 61 (S) | C₁₅H₃₁ | F | H |
| 62 (R) | C₁₆H₃₃ | H | F |
| 63 (S) | C₁₆H₃₃ | H | F |
| 64 (R) | C₁₆H₃₃ | F | H |
| 65 (S) | C₁₆H₃₃ | F | H |

| | | | |
|---|---|---|---|
| * optical isomer | | | |

The phase transition data for these compounds is presented in Table 7 and is as follows:

**Table 7**

| Compound | Phase Transitions/°C |
|---|---|
| 54: | I 88.4 TGB _{A} 87.3 S_{A} 73.4 S_{c}* |
| 55: | I 88.8 TGB _{A} 88.0 S_{A} 72.0 S_{c}* |
| 56: | I 85.8 TGB _{A} 80.8 S_{A} 72.6 S_{c}* |
| 57: | I 87.1 TGB _{A} 82.9 S_{A} 73.5 S_{c}* |
| 58: | I 87.7 TGB _{A} 79.9 S_{c}* |
| 59: | I 86.2 TGB _{A} 78.3 S_{c}* |
| 60: | I 76.6 S_{c}* |
| 61: | I 76.9 S_{c}* |
| 62: | I 85.4 TGB _{A} 78.4 S_{c}* |
| 63: | I 86.1 TGB _{A} 79.4 S_{c}* |
| 64: | I 77.3 S_{c}* |
| 65: | I 78.0 S_{c}* |
| TGB _{A} = Twisted grain boundary A phase. | |

**Table 8**

| Temp/°C | Ps/nC/cm² |
|---|---|
| 74 | 23.6 |
| 72 | 40.5 |
| 70 | 47.6 |
| 65 | 63.0 |
| 60 | 68.7 |
| 55 | 74.8 |
| 50 | 79.0 |
| 45 | 77.5 |
| 40 | 66.5 |

Compound 56: 5.9µm PI parallel cell.

**Table 9**

| Temp/°C | Ps/nC/cm² |
|---|---|
| 75 | 42.2 |
| 70 | 59.0 |
| 65 | 67.7 |
| 60 | 73.5 |
| 55 | 73.0 |
| 50 | 69.0 |

Compound 58: 6.0µm PI parallel cell.

**Table 10**

| Temp/°C | Ps/nC/cm² |
|---|---|
| 76 | 50.5 |
| 74 | 60.0 |
| 72 | 66.0 |
| 70 | 71.0 |
| 65 | 81.0 |
| 60 | 87.0 |

Compound 62: 6.1µm PI parallel cell.

**Table 11**

| Temp/°C | Ps/nC/cm² |
|---|---|
| 73 | 69.4 |
| 72 | 73.1 |
| 70 | 79.1 |
| 65 | 90.0 |
| 63 | 93.2 |
| 60 | 98.1 |
| 55 | 102.5 |
| 53 | 104.3 |
| 50 | 106.0 |
| 45 | 105.0 |
| 43 | 100.4 |
| 40 | 99.6 |

Compound 64: 5.5µm PI parallel cell.

**Table 12**

| Temp/°C | Ps/nC/cm² |
|---|---|
| 81 | 0.3 |
| 80 | 0.5 |
| 75 | - |
| 72 | 1.1 |
| 70 | 1.3 |
| 60 | 1.6 |
| 50 | 1.5 |
| 40 | 1.3 |

Compound 64 10% by weight in H1: 5.1µm PI parallel cell.

## Claims

1. A compound having formula I 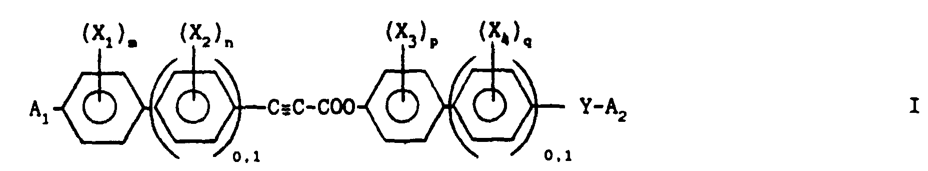 where A₁ is selected from alkyl, alkoxy or alkenyl and contains 3-20 carbon atoms, X₁, X₂, X₃ and X₄ are independently selected from the halogen group; m, n, p and q are independently 0, 1, 2, 3 or 4 such that m+n+p+q ≠ 0; Y is selected from 0 and COO; A₂ is an end group of formula II 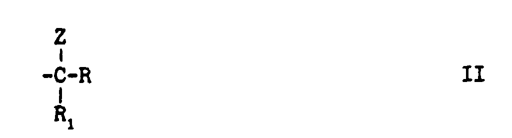 where Z is selected from halogen, CH₃, CN, CF₃, CHF₂; R is a linear or branched alkyl group containing 2-15 carbon atoms or H; R₁ is a linear or branched alkyl group containing 1-5 carbon atoms or H.

2. A compound according to claim 1 wherein A₁ contains 6-16 carbon atoms; R contains 3-10 carbon atoms; Z is CH₃ or CN; R₁ is H; X is F and each of m, n, p and/or q is independently 0, 1 or 2 such that m+n+p+q = 1 or 2.

3. A compound according to claim 1 wherein A₁ contains 9-15 carbon atoms; R contains 5-7 carbon atoms; Z is CH₃ or CN; R₁ is H; X is F and each of m, n, p and/or q is independently 0, 1 or 2 such that m+n+p+q = 1 or 2.

4. A liquid crystal device comprising a layer of liquid crystal material contained between two spaced cell walls each bearing electrode structures and surface treated on facing surfaces to align liquid crystal material molecules, characterised in that the liquid crystal material includes the compound as described in claim 1.

5. A liquid crystal mixture containing any of the compounds of claim 1 and a host material of the following general formula 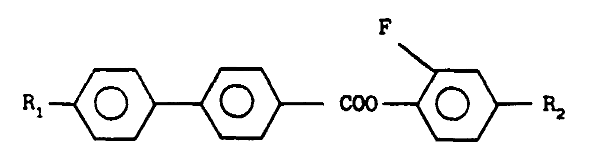 where R₁ and R₂ are independently C₃-C₁₂ alkyl or alkoxy.

6. A liquid crystal mixture containing any of the compounds of claim 1 and a host material of the following general formula 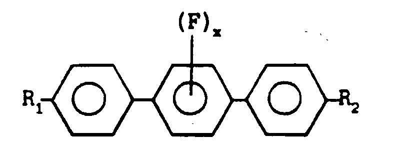 where R₁ and R₂ are independently C₃-C₁₂ alkyl or alkoxy, x is 1 and F may be on any one of the available substitution positions on the phenyl ring specified.

7. A liquid crystal mixture containing any of the compounds of claim 1 and a host material of the following general formula 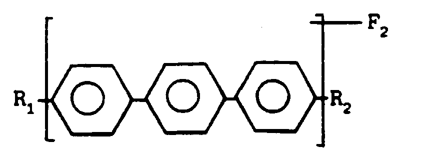 where R₁ and R₂ are independently C₃-C₁₂ alkyl or alkoxy.

8. A liquid crystal mixture containing any of the compounds of claim 1 and a host material of the following general formula 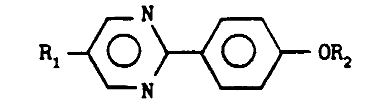 where R₁ is C₃-C₁₂ alkyl and R₂ is given by the general formula (CH₂)ₙ-CHXCH₂CH₃, where n is 1 to 5 and X is CN or Cl.

9. A liquid crystal mixture containing any of the compounds of claim 1 and a host material of the following general formula 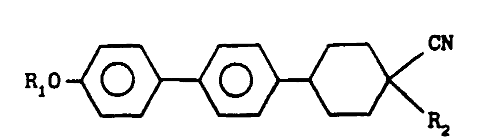 where R₁ and R₂ are independently C₁-C₁₅ alkyl or in the case of R₂ alkoxy.

10. A liquid crystal mixture containing any of the compounds of claim 1 and a host material of the following general formula 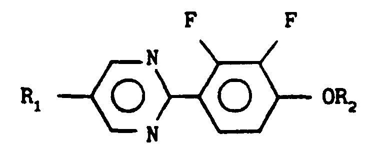 where R₁ and R₂ are independently C₃-C₉ alkyl or in the case of R₁ alkoxy.

11. A device as described in claim 4 including any of the host materials specified in claims 5-10.

## Patentansprüche

1. Verbindungen der Formel I, 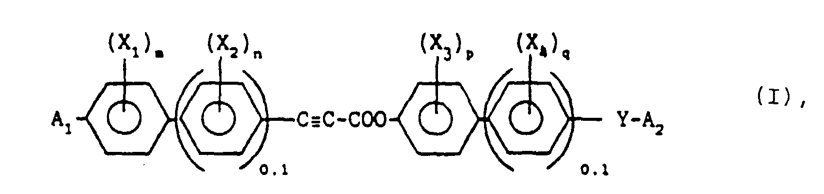 worin bedeuten:
A₁ Alkyl, Alkoxy oder Alkenyl mit jeweils 3 bis 20 C-Atomen,
X₁, X₂, X₃ und X₄ unabhängig ein Halogenatom,
m, n, p und q unabhängig 0, 1, 2, 3 oder 4 mit der Maßgabe, daß m+n+p+q ≠ 0 ist,
Y O oder COO
und
A₂ eine Endgruppe der Formel II 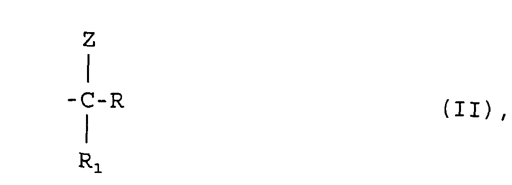 in der bedeuten:
Z ein Halogenatom,
CH₃, CN, CF₃ oder CHF₂,
R H oder geradkettiges oder verzweigtes C₂₋₁₅-Alkyl und
R₁ H oder geradkettiges oder verzweigtes C₁₋₅-Alkyl.

2. Verbindungen nach Anspruch 1, in deren Formel A₁ 6 bis 16 C-Atome und R 3 bis 10 C-Atome aufweisen und Z CH₃ oder CN, R₁ H, X F und m, n, p und/oder q jeweils unabhängig 0, 1 oder 2 darstellen mit der Maßgabe, daß m+n+p+q = 1 oder 2 ist.

3. Verbindungen nach Anspruch 1, in deren Formel A₁ 9 bis 15 C-Atome und R 5 bis 7 C-Atome aufweisen und Z CH₃ oder CN, R₁ H, X F und m, n, p und/oder q jeweils unabhängig 0, 1 oder 2 bedeuten mit der Maßgabe, daß m+n+p+q = 1 oder 2 ist.

4. Flüssigkristallvorrichtung, die eine Schicht aus einem Flüssigkristallmaterial aufweist, die zwischen zwei voneinander beabstandeten Zellenwänden enthalten ist, die jeweils Elektrodenstrukturen aufweisen und auf den einander gegenüberliegenden Oberflächen zur Ausrichtung der Moleküle des Flüssigkristallmaterials oberflächenbehandelt sind, dadurch gekennzeichnet, daß das Flüssigkristallmaterial eine oder mehrere Verbindungen nach Anspruch 1 enthält.

5. Flüssigkristallgemisch, das eine oder mehrere Verbindungen nach Anspruch 1 sowie ein Wirtsmaterial der folgenden allgemeinen Formel enthält, 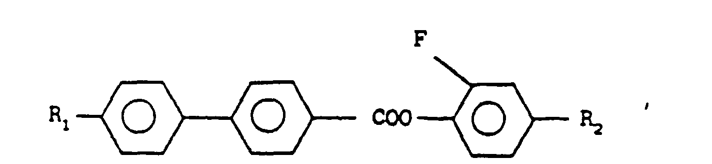 worin R₁ und R₂ unabhängig C₃₋₁₂-Alkyl oder C₃₋₁₂-Alkoxy bedeuten.

6. Flüssigkristallgemisch, das eine oder mehrere Verbindungen nach Anspruch 1 sowie ein Wirtsmaterial der folgenden allgemeinen Formel 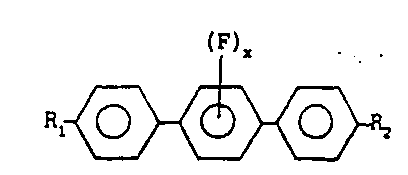 enthält, in der R₁ und R₂ unabhängig C₃₋₁₂-Alkyl oder C₃₋₁₂-Alkoxy bedeuten, x = 1 ist und F sich in einer der möglichen Substituentenstellungen am spezifizierten Phenylring befindet.

7. Flüssigkristallgemisch, das eine oder mehrere Verbindungen nach Anspruch 1 sowie ein Wirtsmaterial der folgenden allgemeinen Formel 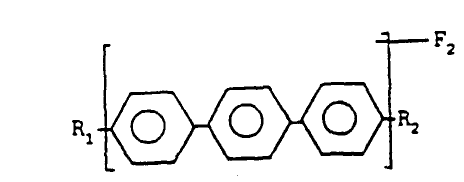 enthält, in der R₁ und R₂ unabhängig C₃₋₁₂-Alkyl oder C₃₋₁₂-Alkoxy bedeuten.

8. Flüssigkristallgemisch, das eine oder mehrere Verbindungen nach Anspruch 1 und ein Wirtsmaterial der folgenden allgemeinen Formel 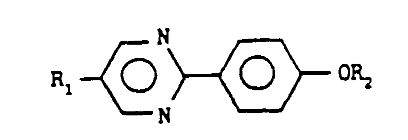 enthält, in der R₁ C₃₋₁₂-Alkyl bedeutet und R₂ durch die allgemeine Formel (CH₂)ₙ-CHXCH₂CH₃ dargestellt wird, in der n 1 bis 5 und X CN oder Cl darstellen.

9. Flüssigkristallgemisch, das eine oder mehrere Verbindungen nach Anspruch 1 und ein Wirtsmaterial der folgenden allgemeinen Formel 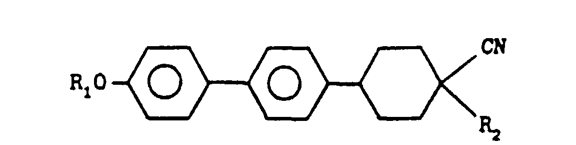 enthält, in der R₁ und R₂ unabhängig C₁₋₁₅-Alkyl oder im Fall von R₂ C₁₋₁₅-Alkoxy bedeuten.

10. Flüssigkristallgemisch, das eine oder mehrere Verbindungen nach Anspruch 1 und ein Wirtsmaterial der folgenden allgemeinen Formel 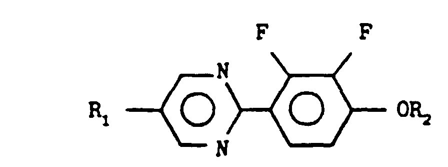 enthält, in der R₁ und R₂ unabhängig C₃₋₉-Alkyl oder im Fall von R₁ C₃₋₉-Alkoxy bedeuten.

11. Vorrichtung nach Anspruch 4, die eines der Wirtsmaterialien nach den Ansprüchen 5 bis 10 enthält.

## Revendications

1. Composé de formule I 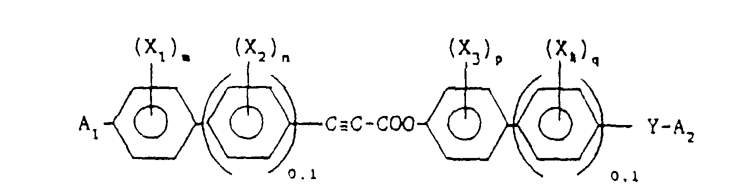 dans laquelle A₁ est choisi entre des groupes alkyle, alkoxy ou alcényle et contient 3 à 20 atomes de carbone, X₁, X₂, X₃ et X₄ sont choisis, indépendamment, dans le groupe des halogènes, m, n, p et q ont indépendamment la valeur 0, 1, 2, 3, ou 4 de manière que la somme m+n+p+q soit différente de 0; Y est choisi entre O et COO ; A₂ est un groupe terminal de formule II 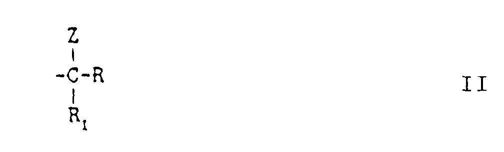 dans laquelle Z est choisi entre un halogène, un groupe CH₃, CN, CF₃, CHF₂ ; R représente un groupe alkyle linéaire ou ramifié contenant de 2 à 15 atomes de carbone ou H ; R₁ représente un groupe alkyle linéaire ou ramifié contenant 1 à 5 atomes de carbone ou H.

2. Composé suivant la revendication 1 dans lequel A₁ contient 6 à 16 atomes de carbone, R contient 3 à 10 atomes de carbone, Z est un groupe CH₃ ou CN ; R₁ représente H ; X représente F et chacun de m, n, p et/ou q a indépendamment la valeur 0, 1 ou 2 de manière que la somme m+n+p+q ait la valeur 1 ou 2.

3. Composé suivant la revendication 1, dans lequel A₁ contient 9 à 15 atomes de carbone ; R contient 5 à 7 atomes de carbone ; Z est un groupe CH₃ ou CN ; R₁ représente H ; X représente F et chacun de m, n, p et/ou q a indépendamment la valeur 0, 1 ou 2 de manière que la somme m+n+p+q soit égale à 1 ou 2.

4. Dispositif à cristaux liquides comprenant une couche de matière à cristaux liquides contenue entre les deux parois espacées d'une cellule portant chacune des structures d'électrodes et traitées en surface sur les faces en vis-à-vis de manière à aligner les molécules de matière à cristaux liquides, caractérisé en ce que la matière à cristaux liquides contient le composé défini dans la revendication 1.

5. Mélange à cristaux liquides contenant l'un quelconque des composés suivant la revendication 1 et une matière hôte de formule générale suivante : 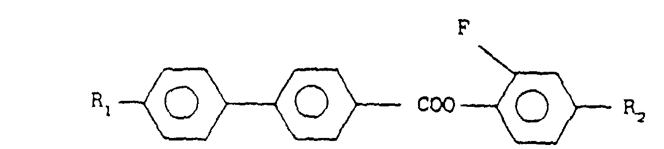 dans laquelle R₁ et R₂ représentent, indépendamment, un groupe alkyle ou alkoxy en C₃ à C₁₂.

6. Mélange à cristaux liquides contenant l'un quelconque des composés suivant la revendication 1 et une matière hôte de formule générale suivante : 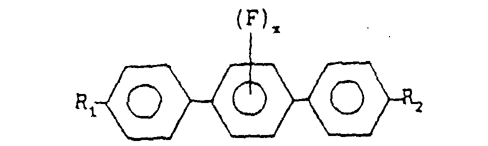 dans laquelle R₁ et R₂ représentent, indépendamment, un groupe alkyle ou alkoxy en C₃ à C₁₂, x est égal à 1 et F peut se trouver dans l'une quelconque des positions disponibles sur le noyau phényle spécifié.

7. Mélange à cristaux liquides contenant l'un quelconque des composés suivant la revendication 1 et une matière hôte de formule générale suivante : 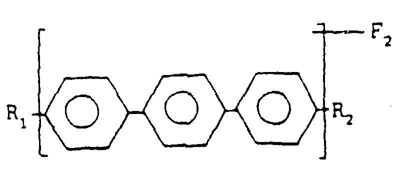 dans laquelle R₁ et R₂ représentent, indépendamment, un groupe alkyle ou alkoxy en C₃ à C₁₂.

8. Mélange à cristaux liquides contenant l'un quelconque des composés suivant la revendication 1 et une matière hôte de formule générale suivante : 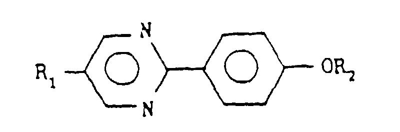 dans laquelle R₁ est un groupe alkyle en C₃ à C₁₂ et R₂ est représenté par la formule générale (CH₂)ₙ-CHXCH₂CH₃, dans laquelle n a une valeur de 1 à 5 et X représente CN ou Cl.

9. Mélange à cristaux liquides contenant l'un quelconque des composés suivant la revendication 1 et une matière hôte de formule générale suivante : 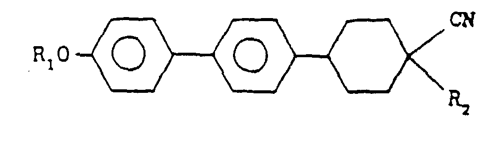 dans laquelle R₁ et R₂ représentent, indépendamment, un groupe alkyle en C₁ à C₁₅ ou, dans le cas de R₂, un groupe alkoxy en C₁ à C₁₅.

10. Mélange à cristaux liquides contenant l'un quelconque des composés suivant la revendication 1 et une matière hôte de formule générale suivante : 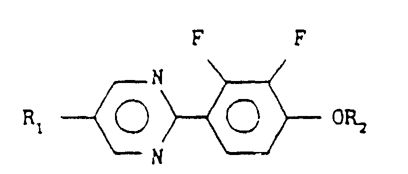 dans laquelle R₁ et R₂ représentent, indépendamment, un groupe alkyle en C₃ à C₉ ou, dans le cas de R₁, un groupe alkoxy en C₃ à C₉.

11. Dispositif suivant la revendication 4, comprenant l'une quelconque des matières hôtes spécifiées dans les revendications 5 à 10.
